# EUROPEAN PATENT APPLICATION

(11) **EP 4 603 078 A1**
(43) Date of publication of application: **20.08.2025**
(21) Application number: 24217356.5
(22) Date of filing: 04.12.2024
(51) Int. Cl.: A61K 8/44, A61K 8/37, A61Q 5/00, A61Q 5/02, A61Q 5/12, A61Q 19/00, A61Q 19/10

(54) **PERSONAL CARE COMPOSITION WITH N-CAPRYLOYL L-GLUTAMINE AND GLYCERYL MONOUNDECYLENATE AS PRESERVATIVE**

(30) Priority: 13.02.2024 IN 202421009600
(71) Applicant: Galaxy Surfactants Ltd., Navi Mumbai 400 703 Maharashtra (IN)
(72) Inventor: KOSHTI, Nirmal, New Jersey, 08854 (US); TALUKDAR, Sukanya, 400615 Thane (W), Maharashtra (IN); JOSHI, Pranali Rajendra, 400705 Navi Mumbai, Maharashtra (IN); SAWANT, Bhagyesh Jagannath, 421306 Kalyan (E), Maharashtra (IN); GOSAVI, Kiran Prakash, 421201 Dombivli East, Maharashtra (IN); SHIRKE, Prashant Prabhakar, 421201 Dombivli East, Maharashtra (IN); SHINDE, Nitesh Suresh, 400701 Navi Mumbai, Maharashtra (IN); SAVLA, Parag Narendra, 421302 Bhiwandi, Maharashtra (IN)
(74) Representative: Müller Schupfner & Partner Patent- und Rechtsanwaltspartnerschaft mbB (Hamburg)

(57) **Abstract**

Personal care compositions with *N*-capryloyl glutamine and glyceryl monoundecylenate as preservative are disclosed. The personal care composition with a combination of *N-*capryloyl L-glutamine (Formula I) 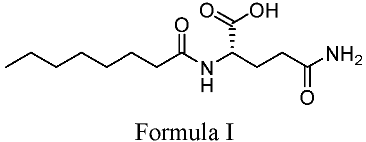 and glyceryl monoundecylenate (Formula II) 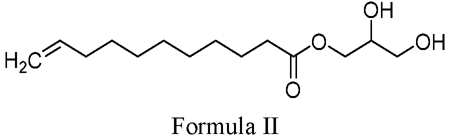 preserve the composition at broad range of pH between 3.0 to 7.0. The components of the preservative composition are manufactured by 'Green' processes that use bio-renewable starting raw materials.

## Description

### Field of Invention:

The present invention relates to a personal care composition. More particularly the present invention relates to a personal care composition comprising *N-*capryloyl L-glutamine (Formula I) and glyceryl mono undecylenate (Formula II) as an anti-microbial preservative along with one or more personal care ingredients.

### Background and prior art of the invention:

Preserving personal care products from microbial contamination/degradation is quite challenging. Most cosmetics products in the form of creams, lotions, gels, shampoos, body-washes and facewashes contain significant amounts of water. This provides a very hospitable environment for microbial growth. In addition to water, other ingredients in such formulations can also be a good source of nutrients to microbes. Another pertinent point to be reckoned here is that the shelf-life of the personal care products and the period that is needed for the consumption of the product after opening the container by the consumer is quite long compared to pharmaceutical products or food products. Unlike pharmaceutical products, personal care products are neither sterilized nor packed in hermetic conditions. Thus, the requirement for preservation of the personal care products is, indeed, quite challenging. This is further compounded by the limited choice of antimicrobials since the available approved antimicrobials are very few and those which have good antimicrobial activity are quite toxic. Consumers want products meant for topical applications to be free from toxic antimicrobials that are used as preservatives. Very effective traditional antimicrobials are implicated in serious toxicity issues to humans as well as to the environment. For example, parabens are implicated in disrupting endocrine system, ultimately linked to breast cancer [(Toxicology and Applied Pharmacology (Vol. 153(1), pp. 12-19 (Nov. 1998), Pharmacology & Toxicology (Vol. 86(3), pp 110-13, March 2000, Journal of Veterinary Medical Science (Vol. 64(3), pp. 227-35 (March 2002, Journal of Applied Toxicology, 24 (3): 167-176, (2004)]. Formaldehyde is classified as Category 3 CMR (carcinogenic, mutagenic, and reproductive toxic) and hence all formaldehyde releasers are under the cloud. This class includes the work-horse preservatives such as DMDM hydantoin, diazolidinyl urea, imidazolidinyl urea and Quaternary-15.

Another class of very effective antimicrobials is 'isothiazolinones'. Methyl and chloromethyl isothiazolinones have been used in home and personal care but these are reported to be neurotoxic and skin sensitizers (Journal of Neuroscience 22 (17): 7408-7416 (2002). The Lancet, Volume 333, Issue 8633, Pages 314-316 (1989). Chloromethyl isothiazolinone (generally abbreviated as CIT) is far more toxic and most of the leading personal care products manufacturers have stopped using it.

Halogenated antimicrobials have their own share of toxicity issues. For example, Triclosan, once a popular antimicrobial for hand sanitizers, is phased out due to its toxicity. It has been implicated in eco-toxicity issues (algae, dolphins). It is reported to be an endocrine disruptor (thyroid function) and is reported to impair cardiac and skeletal muscles. There seems to be a special concern for children who are at higher risk of allergies and the immune systems (Toxicological Sciences, 2009, 107 (1): 56-64, Reproductive Toxicology, Apr 2009, 27(2): 177-185). Companies such as Johnson and Johnson, Proctor & Gamble and Reckitt Benckiser have removed it from hand wash products. Triclosan's ecotoxicity is such a big concern that its usage has been completely banned by the state of Minnesota in the USA (2014). Another anti-fungal agent to meet similar fate is zinc pyrithione which is now completely banned in EU.

Iodopropynyl butyl carbamate, another halogenated antimicrobial, is a contact allergen *(*American Journal of Contact Dermatitis 13(2), 77-79 (2002*).* The presence of iodine in the molecular structure gets it implicated in Goiter and malfunctioning of thyroid gland. It is not allowed in Japan and in European Union (EU) and generally, elsewhere, it is allowed only up to 0.02 % in leave-on products. Similarly, the EU permits usage of methyl dibromo glutaronitrile only up to 0.1 % and that too in only rinse-off products. Another brominated molecule is Bronopol (2-bromo, 2-nitro-1,3 propane diol), very widely used once upon a time, but now it has been restricted to use in cosmetics in Canada. It is involved in allergic reactions as well as generation of *N*-nitroso amines that are known to be carcinogenic. The quaternary ammonium compounds (e.g., cetyl pyridinium chloride, benzethonium chloride, benzalkonium chloride) exhibit good antimicrobial activity but their utility in personal care industry is limited due to incompatibility with anionic surface-active agents. The cationic antimicrobials are completely neutralized by the anionic surface-active agents that are present in large excess in home and personal care formulation (8 to 20 %).

Thus, most of the antimicrobials with phenolic nature or containing halogen (chlorine, bromine, or iodine) are toxic. Also, the big class of `formaldehyde releasing antimicrobials' is being phased out due to the highly toxic nature of formaldehyde. Examples of this class are DMDM hydantoin, imidazolidinyl urea and diazolidinyl urea.

To avoid the above-mentioned toxic antimicrobials, the industry did come up with alternatives antimicrobial preservatives that are largely based on organic acids such as sorbic acid, benzoic acid, dehydroacetic acid, and alcohols such as phenoxyethanol, benzyl alcohol (Cosmetics Directive Annex VI, https://echa.europa.eu/cosmetics-preservatives). In September 2012, French Agency ANSM (Agence Nationale de Sécurité des Médicaments et des produits de santé) raised concerns about the use of Phenoxyethanol as preservative for baby care products due to inadequate safety data and demanded that the upper limit for dose be lowered to 0.4 % from 1.0 % for baby care products meant for children under the age of 3. In 2016, Scientific Community of Consumer Safety (SCCS) finally declared it to be safe up to 1.0 % level for cosmetics. Phenoxyethanol is also reported to depress the central nervous system (Schmuck G, Steffens W, Bomhard E "2-Phenoxyethanol: a neurotoxicant?" Archives of Toxicology. 74 (4-5): 281- 3), (July 2000) and it may cause vomiting and diarrhea, which can lead to dehydration in infants. There are some baby care products that mention on the label about being `free from phenoxyethanol'. (Colgate's baby care brand Tom's of Main, moisturizing lotion). Also, there are baby wipes out in the market that avoid phenoxyethanol as a part of preservation. (Proctor & Gamble, Seventh Generation, The Honest Company, Kimberly Clarke).

Benzyl alcohol, another antimicrobial, suffers from disadvantage of being allergen causing allergic contact dermatitis reactions *(*E. J. Curry and E. M. Warshaw, Dermatitis, 2005; 16 (4): 203-208*)* and strong benzaldehyde (bitter almond) like aroma puts a limitation on its deployment as antimicrobial across the personal care products.

Dehydroacetic acid or its sodium salt is a completely synthetic chemical based on petrochemicals (diketene and malonic acid) has serious limitation of forming colored complexes by chelating traces of iron. It seriously discolors the formulations and hence not a preservative of choice in many cases. Benzoic acid is a petro-chemical based antimicrobial and some formulators try to avoid benzoic acid due to the possibility of it getting oxidized to carcinogenic benzene (Journal of Agricultural and Food Chemistry, 59(24):12975-81(2011)*.*

Avoiding use of petrochemicals and synthetic chemistry that pollutes and generates waste while manufacturing any bulk chemical are the major measures/goals towards saving the planet by slowing down the global warming and the catastrophic consequences that planet is already suffering. Antimicrobial preservatives for personal care products are bulk chemicals, and their usage (and their manufacture) cannot afford to disturb the equilibrium of the planet.

Ideally these need to be made from `renewable source' and they should possess a broad spectrum of antimicrobial activity. These antimicrobials should be biodegradable, non-toxic and compatible with commonly used personal care ingredients. They should be skin-microbiota friendly and organoleptically acceptable.

In view of the limitations of the majority of the existing antimicrobials stemming from the concerns about a) safety to human and ecology (toxic nature), b) use of hazardous chemicals in the manufacturing process, c) use of *non-renewable* petrochemicals, thus there is an urgent need to develop a broad spectrum antimicrobial preservative system that would be a) biodegradable, b) based on `bio-renewable sources' and c) amenable to large scale manufacture.

The preservative system should be 1) efficacious broad spectrum antimicrobial (active against bacteria, yeast and mold) and covering the wide pH range (typical of personal care products), 2) should be biodegradable without harming the ecology, 3) should be manufactured by a process that meets all twelve principles of `Green' chemistry and engineering (Anastas and Warner, Green Chemistry: Theory and Practice New York, Oxford University Press, 1998*)* and 4) that would be totally based on 'renewable' starting materials. Thus, for long term sustainability, all the above 'desired' criteria need to be met with.

### Objectives of the invention

i. It is an objective of the present invention to create an organoleptically acceptable preservative system for personal care compositions that would be based on biodegradable antimicrobials derived from bio-renewable feedstocks.
ii. Another objective of the present invention is to create a broad spectrum antimicrobial preservative system for personal care compositions that are active at pH range of 3.0 to 7.0.
iii. Another objective of the present invention is to create a preservative system for personal care compositions that would be based on antimicrobials which are friendly and supportive to skin microbiota.
iv. Yet another objective of the present invention is to create a preservation system for personal care compositions avoiding use of petrochemicals, hazardous chemicals at any stage of synthesis or processing.
v. Further objective of the present invention is to create an antimicrobial preservative system for personal care compositions that would avoid antimicrobial molecules with any functional group with a potential to be cytotoxic like phenolic moiety, halogens (chlorine, bromine, iodine), 'difficult-to-biodegrade' heterocycles and formaldehyde releasing functionalities etc.
vi. Yet another objective of the present invention is to create a preservation system for personal care compositions avoiding use of hazardous process conditions (e.g., high pressure, high temperature, and dust explosion etc.).

### Summary of the Invention:

In an aspect the present invention relates to a personal care composition comprising:
i. *N-*capryloyl L-glutamine of Formula I;
ii. glyceryl monoundecylenate of Formula II; and 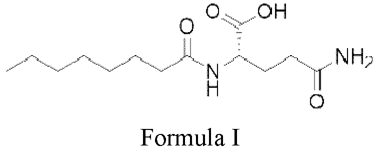 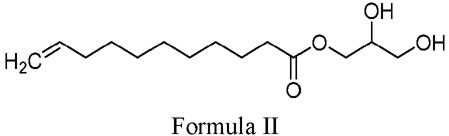
iii. one or more personal care ingredient,
wherein the ratio of the *N-*capryloyl L-glutamine of Formula I and the glyceryl monoundecylenate of Formula II, is from 1:2 to 2:1 by weight.

In an embodiment the personal care composition of the present invention comprises 0.5 to 2.0 weight percentage of the combination *N-*capryloyl L-glutamine of Formula I and glyceryl monoundecylenate of Formula II.

In another embodiment the personal care composition of the present invention has pH ranging from 3.0 to 7.0.

In yet another embodiment the personal care composition is selected from shampoo, body wash, face wash, hair conditioner, hair serum, soap bar, syndet bar, cream, lotion, hygiene wash, deodorant, anti-perspirant and gel.

In yet another embodiment the personal care composition comprises one or more personal care ingredient selected from anionic surfactants, amphoteric surfactants, non-ionic surfactants, cationic surfactants, pearlizers, opacifiers, emollients, anti-inflammatory, anti-microbial preservatives, UV-absorbers, UV-blockers, synthetic and/or natural polymeric conditioners, silicones, gums, rheology modifiers, polymeric rheology modifiers, film formers, vitamins, protein derivatives, anti-acne agents, anti-dandruff agents, moisturizers, humectants, emulsifiers, chelating agents, anti-oxidants, skin care active, hair active, water and mixture thereof.

### Brief description of figures:

Figure 1: Illustrates the H¹ NMR spectrum of *N*-Capryloyl Glutamine of Formula I.
Figure 2: Illustrates the C¹³ NMR spectrum of *N*-Capryloyl Glutamine of Formula I.
Figure 3: Illustrates the effect of the combination of N-Capryloyl Glutamine and glyceryl monoundecylenate against *S. aureus* and *S. epidermidis* through cellular respiration. (SA: set with *S. aureus,* tubes with darker Caps, SE: Set with *S. epidermidis,* tubes with lighter Caps).

### Detailed Description of the Invention:

As discussed in the background section, all those powerful traditional antimicrobials for preservation of personal care products are reported to be toxic to human beings and in some cases, they are reported to be toxic to other forms of life in the environment. All powerful antimicrobials of yesteryears are implicated in toxicity issues ranging from allergies to neurotoxicity to carcinogenicity. Many are reported to disrupt endocrine system. Personal care industry is consciously phasing the toxic antimicrobial preservatives out of products (Examples, chloromethyl isothiazolinone, Triclosan, parabens, DMDM Hydantoin, and Quaternium-15). However, highly efficacious, yet gentle, safe, readily biodegradable and totally bio-renewable alternative preservatives that are produced by 'green' processes are still not available.

In view of this constraint, the personal care industry is trying to use combinations of 'gentle' antimicrobials such as phenoxyethanol, benzyl alcohol, dehydroacetic acid, sorbic acid and benzoic acid, to meet the need of non-toxic preservation of personal care formulations. However, all of these 'gentle' antimicrobials are derived from 'non-renewable' petrochemicals. This is the biggest concern of today. The world has woken up to the damage that has been done by burning fossil fuel, increase in carbon emission (Green House Gases) leading to global warming with catastrophic consequences that the planet is already suffering. The damage that has already been done needs to be reversed. Humans can no longer have the liberty of producing chemicals that are not biodegradable, that are not made from `renewable source' and that are toxic to living systems of the planet. Humans can no longer use hazardous chemicals to synthesize useful ingredients.

In today's time useful functional ingredients of the past lose their usefulness or importance if their manufacturing is accompanied by generation of hazardous waste or hazardous reagents or dangerous process conditions (very high temperature or pressures) of manufacture.

In summary, none of the current antimicrobials, from both toxic and relatively less toxic categories, is derived from 'renewable' source and using principles of 'green' manufacture. (Anastas and Warner, Green Chemistry: Theory and Practice New York, Oxford University Press, 1998*).*

Accordingly, the present invention relates to a personal care composition with N-capryloyl L-glutamine (CAS No 133849-07-7, Formula I) and glyceryl monoundecylenate (CAS No 65684-27-7, Formula II) along with one or more personal care ingredients. Further, the compound of formula I and formula II are manufactured from `renewable sources' using principles of 'Green' chemistry. *N*-capryloyl L-glutamine is manufactured using vegetable derived caprylic acid and fermentation derived L-glutamine using a green process. Similarly, glyceryl monoundecylenate is manufactured by a 'green' process (`Green enzymatic production of glyceryl monoundecylenate using immobilized Candida antartica lipase B', in 'Preparative Biochemistry and Biotechnology' Volume 47, Issue 10, 2017; http://dx.doi.org/10.1080/10826068.2017.1381621) involving esterification of glycerin and undecylenic acid catalyzed by lipase enzyme that results in significant saving of energy (lower temperatures) and faster kinetics (short duration). The enzyme catalyst is recycled several times. The feedstocks for glyceryl monoundecylenate are derived from vegetable oils (undecylenic acid from castor seed oil, *Ricinus communis*)*.* This makes the synergistic combination of *N*-capryloyl L-glutamine and glyceryl monoundecylenate as antimicrobial with RCI (renewable carbon index) of 100 %.

In an embodiment, the present invention relates to a personal care formulation preserved with a combination *N*-capryloyl L-glutamine and glyceryl mono undecylenate.

In another embodiment, the ratio of *N*-capryloyl L-glutamine and glyceryl mono undecylenate is from 2:1 to 1:2 by weight. More particularly, the ratio of *N*-capryloyl L-glutamine and glyceryl mono undecylenate could be any ratio from 2:1 to 1:2 including both mentioned ratios.

In yet another embodiment, the *N*-capryloyl L-glutamine and glyceryl mono undecylenate are incorporated into the personal care composition in the range of 0.25 % to 2.0 % by weight of the personal care composition. More specifically, the *N*-capryloyl L-glutamine and glyceryl mono undecylenate are incorporated into the personal care composition in the range of 0.5 % to 2.0 % by weight of the personal care composition.

*N-*capryloyl L-glutamine and glyceryl monoundecylenate, derived from renewable sources, are compatible with most used ingredients of the personal care compositions. Further, these components are completely biodegradable, as per OECD 301D- Closed Bottle Test, and safe to both human being and environment.

In an embodiment, the *N-*capryloyl L-glutamine and glyceryl monoundecylenate can be used to preserve the personal care composition with broad pH. The personal care composition preserved with the *N-*capryloyl L-glutamine and glyceryl monoundecylenate has pH ranging from 3.0 to 7.0.

In yet another embodiment, the personal care composition of the present invention are selected from but not limited to shampoo, body wash, face wash, hair conditioner, hair serum, soap bar, syndet bar, cream, lotion, hygiene wash, deodorant, anti-perspirant and gel.

In yet further embodiment, the personal care ingredients are selected from surfactants (anionic, amphoteric, zwitterionic, cationic, and non-ionic), bio-surfactants (glycolipids, lipopeptides), pearlizers, emollients, anti-inflammatory, UV-absorbers, UV-blockers, synthetic and/or natural polymeric conditioners, silicones, gums, rheology modifiers, vegetable oils, film formers, vitamins, protein derivatives, anti-acne agents, anti-dandruff agents, moisturizers, humectants, botanicals, emulsifiers and other skin and hair actives.

In yet another embodiment, the *N-*capryloyl L-glutamine and glyceryl monoundecylenate can be suitably used in the personal care compositions along with other known gentle anti-microbial compounds or adjuvants to enhance or boost antimicrobial activity synergistically. Examples of such synthetic yet gentle antimicrobials are sorbic acid, benzoic acid, sodium salt of levulinic acid or phenoxy ethanol. The examples of such adjuvants are ethylhexyl glyceryl ether, caprylyl glycol, 1, 3- propane diol, *N*-acyl glycines, glyceryl caprylate or chelating agents like EDTA that deprive microbes of important metals ions.

In yet another embodiment, the personal care composition comprises 0.5 - 2.0 % w/w of a combination of *N-*capryloyl L-glutamine and glyceryl monoundecylenate in a ratio from 2:1 to 1:2 by weight along with one or more personal care ingredients selected from:
a. 2 to 50 % w/w of surfactants;
b. 0 to 10 % w/w emollients;
c. 0 to 20 % oily substances;
d. 0 to 20 % w/w of UV-absorbers;
e. 0 to 10 % w/w of inorganic UV-blockers;
f. 1 to 10 % w/w of hair and skin benefiting agents;
   0 to 40% w/w of other additives; and
g. 0 to 95 % water.

Preferably, the UV-absorbers are selected from Octocrylene, Avobenzone, Benzophenones, Octyl methoxy cinnamate, Ethyl hexyl triazone, Tinosorb S, Homosalate and Octyl salicylate. The inorganic sunscreens (UV-light blockers) are selected from ZnO, iron oxide, calamine, titanium dioxide and Kaoline.

Preferably, the skin and hair benefitting agents are selected from emollients, humectants, vitamins, synthetic and/or natural polymeric conditioners, vegetable oils, protein derivatives, anti-inflammatory anti-acne agents, anti-dandruff agents, moisturizers, botanicals and other skin and hair actives.

Preferably, the other additives are selected from pearlizers, rheology modifiers, silicones, gums, film formers, emulsifiers, antioxidants and chelating agents.

The preservative composition of the present invention also acts as prebiotic composition for skin microbiota. It selectively supports the growth of skin microbiota, for example *S. epidermis.*

The embodiments of the present invention will now be described in detail below:

### N-Cacryloyl L-Glutamine:

The N Capryloyl L-Glutamine of the personal care composition of present invention is one of the components of said composition. Health and nutritional benefits of L-glutamine are commonly known in art. Human body receives its glutamine from animal products like milk, eggs, meat as well as vegetables like kale, celery, beans, wheat and sprouts. In addition, human body does synthesize L-glutamine. It is needed for muscle building (growth and repair) and is linked to the immune system. It has beneficial effects in patients suffering with Sickle Cell disease. L-Glutamine is industrially manufactured from glucose by fermentation. In the present invention, L-Glutamine and caprylic acid are bonded together via a peptide linkage in *N-*capryloyl L-glutamine (Formula I). Caprylic acid is found in goat milk and hence the name. But it does occur in several vegetable oils in the form of triglycerides. Caprylic acid, being a fatty acid, has beneficial effects to skin and hair through topical application just like any other naturally occurring lipids of hair and stratum corneum. L-Glutamine is a building block of proteins. Proteins in addition to providing structural strength, are involved in specialized functions in the form of enzymes, hormones and antibodies.

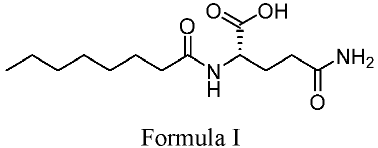

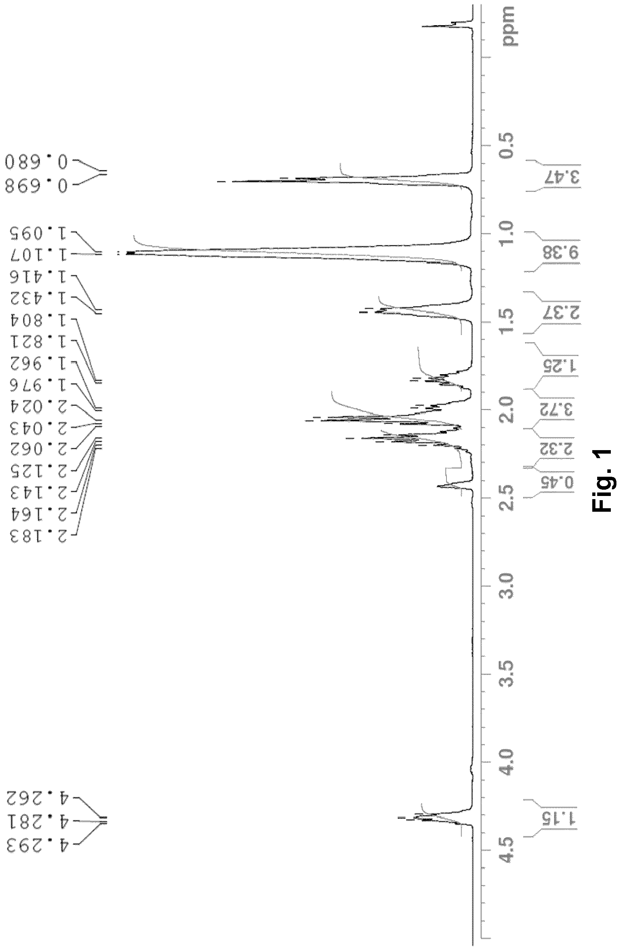

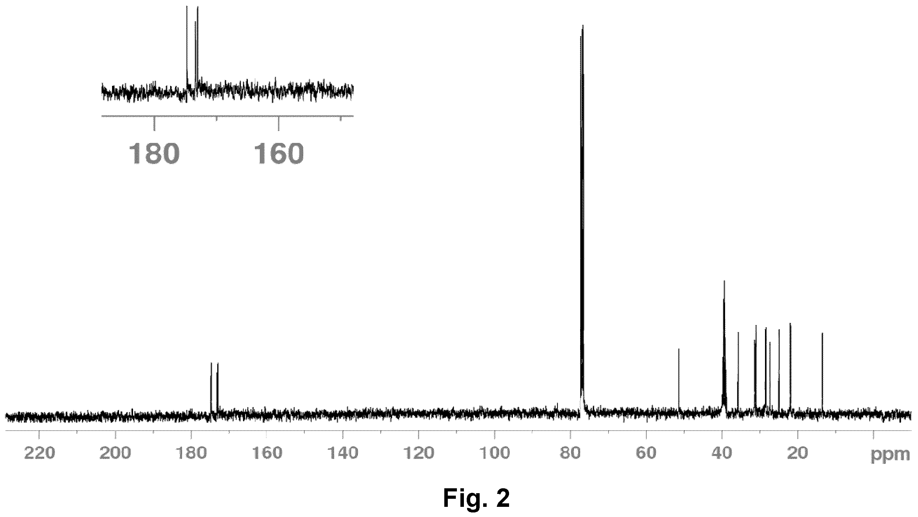

The 'Green' synthesis of *N-*capryloyl L-glutamine is achieved wherein caprylic acid of vegetable origin is converted into its corresponding capryloyl chloride using a heterogeneous biodegradable surfactant catalyst and thionyl chloride (US 9187407). Capryloyl chloride, thus obtained, is reacted with L-Glutamine in aqueous medium in the presence of an inorganic base (Example 1). *N*-Capryloyl L-glutamine is obtained as a white solid with melting point of 120 - 122 °C (99 % yield and with purity 95 %). ¹H NMR reveals a multiplet for the chiral hydrogen at δ 4.26 to 4.29. Interestingly, ¹³C NMR (400 MHz, CDCl₃) shows three distinct signals for carbonyl of amides at δ 172.99 and δ 173.32 and carbonyl of carboxyl at δ 174.73. ((Fig 1 and Fig 2) FT IR shows carbonyl stretch of two amides at 1642 cm⁻¹, 1671 cm⁻¹ and carbonyl stretch of carboxyl groups at1732 cm⁻¹.

### Glyceryl mono undecylenate:

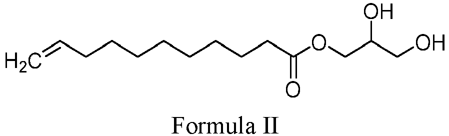

Glyceryl monoundecylenate is another component of the personal care composition of the present invention. Glyceryl monoundecylenate is used as an emulsifier and as an emollient in personal care formulations. As referred in the beginning of this section, (Preparative Biochemistry and Biotechnology, Volume 47, Issue 10, 2017) reported continuous manufacture of glyceryl monoundecylenate using immobilized lipase enzyme using a solvent. Glyceryl monoundecylenate is synthesized with commercially available immobilized lipase, catalyzing the esterification between glycerin and undecylenic acid, but unlike the prior art, the synthesis is accomplished without using any solvent (Example 2). The immobilized catalyst is recycled several times with same kinetics and selectivity (mono ester content > 50 %). It is a light-yellow colored liquid at room temperature (melting point < 14 °C).

### Combination of N-Capryloyl L-Glutamine and Glyceryl Monoundecylenate of the present invention:

*N*-Capryloyl L-glutamine exhibits decent antimicrobial properties against bacteria, yeast and mold (minimum inhibitory concentrations (MICs) listed in Table 1). It can be seen that *N*-Capryloyl L-glutamine is a useful personal-care benefit agent due its excellent inhibitory activity against *Cutibacterium acnes* (MIC, 0.2%) which is involved in proliferation of *Acne vulgaris,* as well as against *Malassezia* yeast (MIC, 0.2%) which is involved in the proliferation of dandruff. It should also be noted that *N*-capryloyl L-glutamine has moderate activity against sweat degrading gram positive residents *Staphylococcus hominis* (MIC, 0.6 %) *Micrococcus luteus* (MIC 0.5 %) and *Corynebacterium xerosis* (MIC, 0.5%) of axillary vault region of human. This property is useful in personal care composition designed to control the generation of body odor.

Similarly, glyceryl monoundecylenate exhibits very good activity against *Malassezia* yeast (MIC, 0.2 %) that is implicated in the proliferation of dandruff; however, it shows weak action against the rest of the test organisms (Table 1) that include G -ve, G +ve bacteria, and mold. Though glyceryl monoundecylenate shows weak activity against mold and most of bacteria including commensal *Staphylococcus epidermidis,* (MIC, 0.9%), however, it is found to inhibit infectious *Staphylococcus aureus* at very low concentration (MIC, 0.1 %). Thus, *N*-capryloyl L-glutamine and glyceryl monoundecylenate are gentle antimicrobials and combining them seems to result in synergy (third column in Table 1) as can be seen in MIC numbers against *Malassezia, Candida, Corynebacterium xerosis, Micrococcus luteus and Staphylococcus hominis* (the last three G +ve are responsible for sweat degradation and malodour generation in armpits) and against highly infectious *Staphylococcus aureus.*

**Table 1: Minimum Inhibitory Concentrations of N-capryloyl L-glutamine and glyceryl monoundecylenate individual and combination.**

| | **GMU*** | **CG**** | **GMU*:CG* * (1:1 by weight)** |
|---|---|---|---|
| **Microorganisms** | **MIC in %** | **MIC in %** | **MIC in %** |
| Gram positive | | | |
| *Staphylococcus aureus* ATCC 6538 | 0.1 | 0.3 | 0.1 |
| *Cutibacterium acnes* ATCC 6919 | 0.9 | 0.2 | 0.5 |
| *Staphylococcus epidermidis* NCIM 2493 | 1 | 0.9 | 1 |
| *Staphylococcus hominis* MTCC *8980* | 0.7 | 0.6 | 0.3 |
| *Micrococcus luteus* NCIM 2103 | 0.5 | 0.5 | 0.3 |
| *Corynebacterium xerosis* ATCC 373 | 0.5 | 0.5 | 0.3 |

| Gram variable | | | |
|---|---|---|---|
| *Gardnerella vaginalis* ATCC 14018 | 0.3 | 0.6 | 0.5 |

| Gram negative | | | |
|---|---|---|---|
| *Escherichia coli* ATCC 8739 | 1 | 0.7 | 1 |
| *Pseudomonas aeruginosa* ATCC 15442 | 1 | 0.7 | 0.9 |
| *Burkholderia cepacia* ATCC 25416 | 1 | 1 | 1 |

| Yeast | | | |
|---|---|---|---|
| *Candida albicans* ATCC 10231 | 1 | 0.6 | 0.4 |
| *Candida glabrata* ATCC 66032 | 0.4 | 0.6 | 0.2 |
| *Malassezia furfur* ATCC 14521 | 0.2 | 0.3 | 0.1 |
| *Malassezia restricta ATCC MYA 4611* | 0.2 | 0.4 | 0.2 |

| Mold | | | |
|---|---|---|---|
| *Aspergillus niger* ATCC 16404 | 0.8 | 1 | 1 |

| | | | |
|---|---|---|---|
| *GMU - Glyceryl monoundecylenate **CG - N Capryloyl L-glutamine | | | |

The synergy of the combination of the *N-*capryloyl L-glutamine and the glyceryl monoundecylenate is also tested out in preservation of the personal care composition as per present invention. The cleanser system in Example 3 represents surfactant combination (anionic surfactant, sodium acyl glutamate and sodium acyl isethionate with amphoteric surfactant alkyl aminopropyl betaine) which is typical personal cleansing formulation (about 16% active). The combination of *N*-capryloyl L-glutamine and glyceryl monoundecylenate is evaluated for preservation efficacy of this typical personal care formulation (Example 3, Table 2a) and for the synergy between them at pH, 5.5 (skin pH) and 7.0 (neutral). When these two antimicrobials are deployed alone at 2 % in this surfactant composition, both *N*-capryloyl L-glutamine as well as glyceryl monoundecylenate individually preserve the composition (challenge test results in Table 2b in Example 3) at both acidic and neutral pH.

However, they fail to pass the challenge test when deployed at 1.5 % level individually in the same formulation. Surprisingly, when used as a combination at 1.5 % of the total cleansing composition (1:1 ratio by weight, with 0.75 % each, totaling up to 1.5 %) then the combination passes the preservation efficacy test at both pHs. This clearly shows the synergy between the *N-*capryloyl L-glutamine and glyceryl monoundecylenate in terms of covering the broad spectrum of microbes and the broad range of pH.

Interestingly, synergy is visible in combination with 1:2 to 2:1 ratio at 1.5 % concentration at both pH (entries 11,12,13, &14 in Table 2b). Stretching the ratio of this combination beyond this range weakens the synergy. The skewed combinations of the two components beyond the limits of 2:1 to 1:2 fail to preserve personal care formulations unless higher amount (%) is used, thus taking effective preservation level to the dosage level of individual antimicrobial employed in entries 1, 2 or 5 or 6 of Table 2b. Therefore, the ratios of the components of *N-*capryloyl L-glutamine and the glyceryl monoundecylenate as per present invention are critical to provide the optimum preservation efficacy of the personal care composition. The components of *N-*capryloyl L-glutamine and the glyceryl monoundecylenate of present invention when used in the ratio of 3:1 or 1:3 would not preserve the formulations effectively. Further when formulating the personal care formulation with the preservative system of the present invention beyond the ratio 2:1 or 1:2 may result in incompatibility related challenges.

In an embodiment several personal care compositions of various types have been tested for the preservation efficacy of the combination of *N-*capryloyl L-glutamine and glyceryl monoundecylenate using PCPC (Personal Care Products Council, Washington DC, formerly known as CTFA) protocol (`Evaluation of preservatives to protect cosmetics' by D. Orth in Cosmetics and Toiletries, March 91). The initial inoculation level of microbes for this study is 10⁷ cfu / mL for bacteria and 10⁶ cfu/mL for yeast and mold in the formulation. Both 'rinse-off and 'leave-on' formulations (Examples 5 to 10) passed the challenge tests.

The personal care composition of the present invention (Examples 5 to 13 and Example 15) comprise combination of *N-*capryloyl L-glutamine and glyceryl monoundecylenate as antimicrobial preservative in amount of 0.5 to 2.0 % w/w and one or more ingredients selected from anionic surfactants, amphoteric surfactants, zwitterionic surfactants, cationic surfactants, non-ionic surfactants, biosurfactants, pearlizers, emollients, anti-inflammatory, UV-absorbers, UV-blockers, synthetic and/or natural polymeric conditioners, silicones, gums, rheology modifiers, vegetable oils, film formers, vitamins, protein derivatives, anti-acne agents, anti-dandruff agents, moisturizers, humectants, botanicals, emulsifiers and other skin and hair actives.

Biodegradable and semi-synthetic/semi natural anti-dandruff /anti-acne agents like *N-*capryloyl glycine (CAS No. 14246-53-8) and N-undecylenoyl glycine (CAS No. 54301-26-7) can be used in conjunction with the natural antimicrobials of the present application. Since the *N*-acyl glycines exhibit good antimicrobial activity (against yeast and bacteria), their incorporation in the compositions given in Examples is avoided. This ensures that the preservation efficacy is entirely derived from the antimicrobials of the present invention.

The body wash composition of Example 5 is made at pH 5.5 and at pH 7.0 and are preserved with 1.5 % concentration with the *N-*capryloyl L glutamine and glyceryl monoundecylenate (1:1 by weight). The body wash of Example 5 is rechallenged to demonstrate successful preservation efficacy. Various types of personal care compositions (Examples 6 to 13, and 15) with varying amount of water and pH ranging between 3.0 to 7.0, have been preserved with the combination of two 'green' antimicrobials of the present invention. In rinse-off cleansing compositions, body wash (Examples 5 and 7) and shampoo (Example 6) are at pH of 5.5 and 6.0 respectively whereas anti-acne facial cleanser (Example 8) and feminine intimate hygiene wash (Example 9) are at pH 4.5 and 3.5. The body wash composition with pH 5.5 is rechallenged after keeping it at 40 °C for a month and it passes the re-challenge test. The intimate hygiene wash of Example 9 uses the blend of the two antimicrobials (described in Example 14) as a convenient way of incorporating this preservation system in personal care formulations. This synergistic combination of two components also preserves the personal care composition at lower concentrations (0.5 to 1.2 % of the total composition) as exemplified in Example 10, 11 and 12 wherein both antimicrobials are employed at 0.25 to 0.6 % level in the combination for preservation of personal care composition. In addition to being an effective preservation system (Example 11), the combination of the two antimicrobials is effective in odor control by acting on the *Staphylococcus hominis, Corynebacterium xerosis* and *Micrococcus luteus* as shown in column 3 of Table 1. Similar synergy is seen for this combination against yeast *Candida albicans, Candida glabrata* and bacterium *Gardnerella vaginalis* which are involved in problems with feminine intimate hygiene (Example 9). Other personal care benefits like skin hygiene and scalp hygiene are derived from its activity against *Cutibacterium acnes* and *Malassezia* yeast in acne and dandruff control respectively. Skin hygiene cleansing bar with odor control based on sodium cocoyl isethionate (with pH similar to skin) is described in Example 13.

### N-Capryloyl L-glutamine and glyceryl monoundecylenate as prebiotic ingredients:

*Staphylococcus epidermidis* and *Staphylococcus aureus* are part of skin microbiota. *Staphylococcus epidermidis* is innocuous.

In natural environment *Staphylococcus epidermidis* is symbiont and prevents colonization of highly infectious *Staphylococcus aureus* which is highly contagious and causes serious diseases. *Staphylococcus aureus* spreads by direct contact with an infected person, by using a contaminated object or by inhaling infected droplets dispersed by sneezing or coughing. Skin infections from *Staphylococcus aureus* are common, and the bacterium can spread through the blood stream and infect distant organs. Skin infections may cause blisters, abscesses (furuncles), redness and swelling (*Cellulitis*) in the infected area. *Staphylococcus aureus* infects hair roots (*Folliculitis*) by developing a small pimple at the base of the hair. It also causes fluid filled blisters (*Impetigo*) on skin that burst. *Staphylococcus aureus* also causes *Mastitis* of breast after 1 to 4 weeks after the delivery, which may include cellulitis and abscesses. The area around the nipple becomes red and painful. Abscesses often release large numbers of bacteria into the mother's milk. The bacteria may then infect the nursing infant. In children, *S. aureus* causes epidermal necrolysis that results in peeling of skin. Toxins of *S. aureus* also result in food poisoning and 'toxic shock syndrome'. Some species are also known to release coagulase enzyme that results in blood clotting. Once it enters blood then it results in osteomyelitis or pneumonia. Also, many strains that originate from hospital or from hospital staff are antibiotic resistant and hence any antimicrobial that has decent activity against *Staphylococcus aureus* is welcome especially for topical application where resident *Staphylococcus aureus* can be the source of not only skin/hair infections but other systemic infections and subsequent human to human spreading of the infections.

Thus, *Staphylococcus aureus* of skin microbiota is pathogenic and in contrast to this, the *Staphylococcus epidermidis* is commensal. The commensal *S. epidermidis* is involved in many host benefitting actions. It prevents colonization of *S. aureus* by secreting serine protease that destroys the biofilm of *S. aureus* (Iwase et al.; Nature, Letters Vol 465, p 246-250 (2010*)).* Modulins produced by *S. epidermidis,* along with host produced AMPs, selectively inhibit pathogens like *S. aureus and Streptococcus. S. epidermidis* is also involved in immunity pathway signaling and in anti-inflammatory activity by secreting lipoteichoic acid. (E. A. Grice and J. A. Segre, Nature Reviews: Microbiology, Volume 9, 243-253 (2011*)).*

Thus, the work by the scientific community over a decade (Julia Segre et al.; Nature Reviews, vol 116, p143-155, (2018)), has established that *S. epidermidis* is one of the major commensals that prevents colonization of pathogenic *Staphylococcus aureus* by three distinct pathways, 1) by destroying the biofilm of *S. aureus* by secreting serine protease glutamyl endopeptidase, 2) by signaling the immune cells and subsequently generating antimicrobial peptides, and 3) by generating the antibiotics, lantibiotics, that work synergistically with Cathilicidine.

The inventors of the present invention surprisingly found that the synergistic combination of 'green' antimicrobials, N-capryloyl L-glutamine and glyceryl monoundecylenate is not only effective for delivering personal care benefits (preservation, anti-dandruff, anti-acne, anti-malodor etc.) but it is also supports (prebiotic) the skin microbiota in being aggressive against pathogenic *Staphylococcus aureus* and being gentle towards symbiont *Staphylococcus epidermidis* which competes with infectious *S. aureus* and prevents it from colonizing on human skin (Table 1). At skin pH of 5.5, the minimum inhibitory concentration of glyceryl mono undecylenate is 0.1 % against *Staphylococcus aureus* whereas against *Staphylococcus epidermidis* it is 1.0 %. Similarly, *N-*capryloyl L-glutamine has minimum inhibitory concentration of 0.3 % against infectious *Staphylococcus aureus* whereas against symbiont *Staphylococcus epidermidis* it is 1.0 %. Interestingly, by combining both antimicrobials in equal-mass ratio, the minimum inhibitory concentration of this 1:1 combination (by weight) remains at 0.1 % against *Staphylococcus aureus* and 1.0 % at *Staphylococcus epidermidis.*

The strong action of the combination, *N*-capryloyl L-glutamine and glyceryl monoundecylenate, of the present invention against infectious *Staphylococcus aureus* and relatively much weaker activity against commensal *Staphylococcus epidermidis* is verified individually as well as in 1:1 combination (by weight) using redox dye Triphenyl Tetrazolium Chloride (TTC) which goes to its reduced form, red colored triphenyl formazan (equation below) as a result of reductive enzymes generated in the process of cellular respiration of microbes (Bulat H, 'Reduction processes in living tissue, formazan, tetrazolium salts and their importance as reduction oxidation indicators in resting seed ; Proceedings of the International Seed Testing Association, V 26, P 686-696, 1961*).*

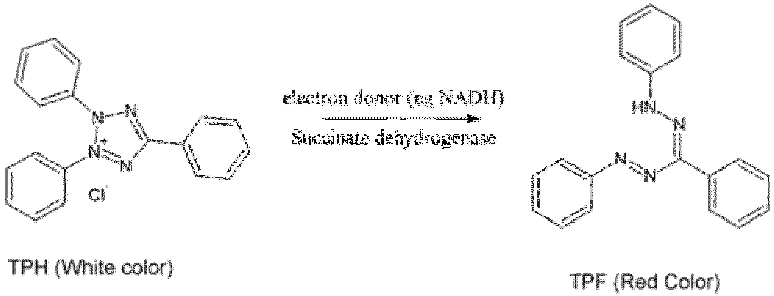

As explained in Example 4, the 'control' set is prepared with PBS (Phosphate Buffer Saline) + Tryptic Soy Broth (TSB) + bacterium (*S*. *aureus or S. epidermidis)* + TTC (Triphenyl Tetrazolium Chloride).

The 'Test' set is prepared with three different concentrations, (combination of N-capryloyl L-glutamine and glyceryl monoundecylenate, 1:1 by weight), 0.1 %, 0.3 % and 0.5 % (The test substance + PBS + TSB + bacterium (*S*. *aureus or S. epidermidis*) + TTC). Twenty-four-hour old cultures with concentration of around 1×10⁷ are used. After preparing the two sets of tubes (as given in the Table 3a and 3b), one with *Staphylococcus aureus* and the other with *Staphylococcus epidermidis,* are vortexed and incubated for 24 h at 35°C. The 'Control' sets (PBS + TSB + bacterium + TTC) turn pinkish red after 24 h incubation for both organisms, *Staphylococcus aureus* as well as *Staphylococcus epidermidis* due to cellular respiration.

The 'Test' set (Test substance with varying concentrations + PBS + TSB + bacterium + TTC) shows decreasing intensity of pink color with increasing the concentration of the test substance.

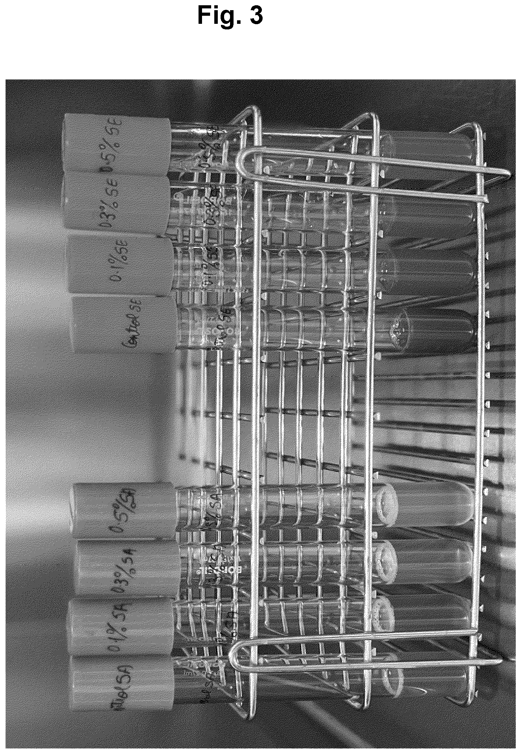

The tubes of 'Test' set (red caps) for *Staphylococcus aureus,* turn red at concentration of 0.1% and slightly pinkish at 0.3 % of test substance whereas it is practically colorless at 0.5 % concentration indicating virtually nil to negligible cellular respiration to effect in 24 h incubation. The 'Test' set *for Staphylococcus epidermidis (yellow caps)* shows formation of red color at the end of 24 h incubation which gradually fades in going from 0.1 % to 0.5 % concentration of the test substance (Fig 3, Example 4). As expected, the red color deepens with the increasing incubation period from 16 h to 24 h to 48 h and so on). However, the difference between the two sets corresponding to two organisms remains stark.

The intensity of red color for *S. epidermidis* is stronger compared to *S. aureus* for the corresponding concentrations. At 0.5 % the tube with *S. aureus* is colorless *and S. epidermidis* is measurable, pink color, thus, indicating that the test substance (1:1 mixture of N-capryloyl L-glutamine and glyceryl mono undecylenate) exhibits higher antimicrobial activity towards *S aureus* and significantly lesser activity towards *S. epidermidis.* These tubes (after twenty-four hours of incubation) are further diluted with Tryptone Azolectin Tween broth (TAT) and plated on Modified Letheen Agar (MLA) plates. The plates are incubated at 35 °C for 48 h and the bacterial counts (cfu/ml) are determined (Table 3c, Example 4).

From the measurement of the bacterial count, it can be seen that the count of the 'control' tube after plating is close to the original concentration of the microbes. Interestingly, the test substance shows one log reduction for the tube with 0.1 % concentration, and 2 log reduction for the tubes with 0.5 % for *Staphylococcus aureus* whereas the three different concentrations of the test substance (of 0.1, 0.3 and 0.5 %) show very weak activity against *Staphylococcus epidermidis.*

It is also important to note that the biofilm forming property of infectious *Staphylococcus aureus* is supported by *Propionibacterium acnes* that produces coproporphyrin III to promote aggregation of *Staphylococcus aureus* (Julia Segre et al. Nature Reviews, vol 116, p143-155, (2018)). The antimicrobials combination of present invention shows a good activity against *Cutibacterium acnes* (Table 1), and this indirectly helps prevent formation of biofilms by *Staphylococcus aureus.* Thus, the prebiotic combination of N-capryloyl L-glutamine and glyceryl mono undecylenate of the present invention are capable of generating a probiotic situation on the skin by creating higher population of commensal *S. epidermidis* which in turn will suppress pathogenic *S. aureus* by way of generating antimicrobial peptides as well as by generating film destroying enzyme and by biosynthesizing antibiotics. Thus, the combination of *N-*capryloyl L-glutamine and glyceryl monoundecylenate of the present invention has huge potential to create a prebiotic effect via personal care formulations, particularly via 'leave-on' applications. Thus, the synergistic combination of antimicrobial preservatives of the present invention is a biodegradable, bio-sourced, and prebiotic system for skin with other additional benefits derived from it like anti-dandruff, anti-acne and anti-malodor activity.

### Advantages of the Invention

### 1) Bio-renewable feedstocks, green process of manufacture

Both antimicrobials of the present invention, N Capryloyl L-glutamine and glyceryl monoundecylenate, are derived from `bio-renewable sources' namely caprylic acid, L-glutamine, undecylenic acid and glycerine. Caprylic acid, undecylenic acid and glycerin are derived from vegetable oils and L-glutamine is manufactured by fermentation of glucose. Glyceryl monoundecylenate is synthesized by esterification of glycerine and undecylenic acid using recyclable immobilized lipase enzyme.

The syntheses of both *N*-capryloyl L-glutamine and glyceryl monoundecylenate meet all twelve principles of 'Green' chemistry. The process of making *N*-capryloyl L-glutamine doesn't involve any hazardous reagents or dangerous reaction conditions. It doesn't produce any wastage that needs to be disposed of. The yields of the processes are quantitative and there is no wastage at any stage.

### 2) Biodegradable with 100 % RCI

The combination of these two antimicrobials in 2:1 to 1:2 ratio results in naturality index of 100 %. Both *N-*Capryloyl L-glutamine and glyceryl monoundecylenate are biodegradable and degraded products are harmless to the environment. The degraded products of fatty acids, glycerine and L-glutamine are well-metabolized by living systems.

### 3) Synergistic combination results in a broad spectrum of antimicrobial activity over a broad range of pH

The combination of N-capryloyl L-glutamine and glyceryl monoundecylenate of the present invention is effective over a wide range of pH (3.0 to 7.0) that covers all personal care applications.

### 4) Prebiotic: Skin-microbiota supportive

The synergistic combination of gentle antimicrobials of the present invention is active against infectious *Staphylococcus aureus* and gentle on commensal *Staphylococcus epidermidis.* This synergistic combination helps commensal *Staphylococcus epidermidis* prevent the colonization of infectious *Staphylococcus aureus.* Thus, using the right combination of these two gentle antimicrobials, it is possible to create personal care formulations with skin microbiome friendly /supportive properties (Park et al. US Pat Appl, 2023 /0381094 A1).

### 5) Several personal care benefits other than preservation

The gentle antimicrobials of the present invention offer several benefits in addition to preservation. They exhibit very good activity against microbes that are involved in proliferation of dandruff and acne, generation of mal-odor and feminine intimate hygiene. The preservative system of the present invention comprising N-capryloyl L-glutamine and glyceryl monoundecylenate, is based on linear fatty acid type molecules that blend well with skin lipids. Its decent antimicrobial activity is useful for preventing hyperproliferation (*Cutibacterium acnes, Malassezia, and Candida*) of skin microbiota. Thus, it serves as a 'Derma purifier' overall contributing to skin and scalp hygiene via leave-on personal care products.

The preservative system of the present invention comprising, N-capryloyl L-glutamine and glyceryl monoundecylenate, is active against the microbiota of axillary vault that mainly comprises of *Staphylococci and Corynebacteria.* Thus, in addition to being useful as a preservative system of personal care composition, it can control the microbes like *Staphylococcus hominis, Staphylococcus epidermidis and Corynebacteria xerosis* that degrade secretions of apocrine glands (sweat) to generate malodorous molecules.

### 6) Gentle yet effective sustainable alternative to current toxic anti-microbials

The personal care composition of the present invention comprising *N*-capryloyl L-glutamine and glyceryl mono undecylenate, is an effective preservative alternative to the toxic (endocrine systems disruptors or neurotoxic to humans and ecology) antimicrobials that continue to destroy our planet (mentioned in the background section).

### 7) Free from any potential toxic functionality

The components, N-capryloyl L-glutamine and glyceryl mono undecylenate, don't contain any inherent structural features such as phenolic moiety, halogens or any formaldehyde releasing functionality etc. that would be expected to be cytotoxic.

### 8) Compatible with other personal care ingredients

The components N-capryloyl L-glutamine and glyceryl mono undecylenate of the present invention are compatible with all commonly used personal care ingredients.

### 9) Natural alternative to current toxic and fossil fuel based antimicrobial preservatives

As explained in the background that the arsenal of effective yet non-toxic antimicrobials has shrunk significantly to a very few gentle molecules with `limited antimicrobial activity' like phenoxy ethanol, benzyl alcohol, benzoic acid, sorbic acid, dehydroacetic acid etc. Though these are relatively far less toxic compared to halogen containing antimicrobials, the major disadvantage they suffer from is their origin. These are not made from bio-renewable raw materials. All are synthesized from fossil fuel/petrochemicals (phenol, ethylene oxide, toluene, diketene, malonic acid and trans-butenal etc.). In view of this fact, the addition of *N*-capryloyl L-glutamine to the current limited arsenal of gentle natural antimicrobials opens several options of synergistic combinations for combinatorial approach for preservation.

### Examples:

The present invention is now described by way of non-limiting illustrative examples. The details of the invention provided in the following examples are given by way of illustration only and should not be construed to limit the scope of the present invention.

Bio-surfactant, 'Sophorolipids' is procured from Galaxy Surfactants Limited, Mumbai, India. It is made by fermentation process with oleic acid and glucose as the carbon sources using the yeast, *Candida bombicola.* It is an aqueous solution (50 %) and free from any externally added antimicrobial preservatives.

All other aqueous surfactants are used from in-house production (Galaxy Surfactants Ltd, India) to ensure that these do not contain any antimicrobial preservative. Examples of such aqueous surfactants are cocamidopropyl betaine, lauryl polyglucoside, sodium cocoyl taurate and sodium lauroyl sarcosinate and sodium lauroyl glutamate. Preservative free blend of sodium lauroyl methyl isethionate and sodium lauroyl methyl taurate (Galsoft TLMI 12) in aqueous solution form is provided by Galaxy Surfactants Ltd.

Sodium cocoyl isethionate (85 %) in powder form (Galsoft SCI -85) is supplied by Galaxy Surfactants Ltd. A blend of sodium cocoyl glutamate and sodium cocoyl isethionate (2:1) in powder form (Galsoft GLI 21) is procured from Galaxy surfactants Ltd. 1, 3-propanediol distearate is prepared according to the literature procedure (US 9,833,395). Aculyn 60 (PEG 150 distearate) is procured from Dow. Polyquaternium-7 is water-soluble cationic polymer (9 % solution) without any preservative. Dimethiocone is procured from Dow. All the chemicals are procured from the suppliers or their distributors.

The MIC (minimum inhibitory concentrations) of *N*-capryloyl L-glutamine and glyceryl monoundecylenate and 1:1 mixture (by weight) of the two antimicrobials against various microbes are determined as per PCPC (Personal Care Product Council, Washington, USA) protocol.

### Example 1: Synthesis of N-capryloyl L-glutamine

To a stirred solution of L-Glutamine under nitrogen blanket (42 g, 0.28 mol) in water (352 ml) with pH of 10.5 adjusted with NaOH (48%) capryloyl chloride is added (44.5 g, 0.27 mol) while maintaining the pH of the reaction mass between 10.5 to 11.5 with simultaneous addition of sodium hydroxide (48 % solution, 46 g, 0.54 mol) solution at 20 - 30°C. After completion of the addition of capryloyl chloride, the reaction mass is further stirred for 2 h. The reaction mass is acidified with concentrated hydrochloride acid to pH 1.5 and the precipitated solid is filtered and washed with water to remove the mineral acidity. Drying of wet cake yielded *N*-capryloyl L-glutamine as white solid (74 g, 99 % yield).

FTIR: (as such) 1732, 1671, 1642, 1621, 3437 and 3355, 3309 cm⁻¹

Melting point: 120 - 122°C, Optical rotation: -3.463°, Concentration = 4% in methanol at 20°C and 589 nm.

¹H NMR (400 MHz, CDCl₃) δ: 7.0 (s,1H), 6.8 (s,1H), 5.88 (s, 1H), 4.2 (m, 1H), 2.1 - 2.0 (m, 6H), 1.4 (m, 2H), 1.1 (s, 8H), 0.7 (t, 3H).

¹³C NMR (100 MHz, CDCl₃) δ: 174.73, 173.32, 172.99, 51.33, 35.66, 31.28, 28.53, 27.36, 24.95, 21.90, 13.44.

### Example 2: Synthesis of glyceryl monoundecylenate

A mixture of undecylenic acid (100 g, 0.54 gmol), glycerine (150 g, 1.62 gmol) and immobilized lipase (*Candida antartica*) catalyst (5 g, Addzyme 015, Advanced Enzymes, India) is stirred under nitrogen and reduced pressure of 20 mmHg at 65-70 °C. The progress of the reaction is monitored by the drop in the acid value of the reaction mixture. The reaction mixture is cooled down to room temperature after the acid value of less than 2 was achieved (9 h) and the catalyst was removed by filtration. The filtered product is allowed to phase-separate, and the lower level of glycerin was removed. Recovered glycerin and the isolated catalyst is recycled for the next batch and thus, seven additional experiments are performed by recycling the catalyst.
Sap value: 230, Acid value: 1.0, glycerin content: 3.0 %, Iodine value =100. Color: 35 APHA
FTIR: 1737 (carbonyl), 2857, 2928 (C-H stretch) and 3405 (OH) cm⁻¹
Gas Chromatography: Agilent Gas chromatograph (model number 7890B) coupled with FID. Capillary Column: HP-5MS UI (30m × 0.25mm × 0.25 micron), Injector port temp: 250 °C, Detector port temp: 300 °C, carrier gas: Helium 1 cc/min, column temperature programming: 30 °C to 300 °C with 15 °C. Total run time 35 min.

Gas chromatographic analysis in terms of monoester content for seven cycles produced by recycling the same catalyst of the first cycle which above 50 % (50-55%).

### Example 3: Preservation efficacy of N-capryloyl L-Glutamine (CG) and Glyceryl monoundecylenate (GMU) and combination of both in ratio of 1:1 in a cleansing surfactant composition.

Using the typical surfactant composition two sets are prepared at different pHs, 5.5 and 7.0. Antimicrobials individually and in combination are tested for preservation efficacy.

**Table 2a: Cleansing formulations I and II with pH 5.5 and 7.0 respectively**

| **Ingredients** | **(I) w/w%** | **(II) w/w%** |
|---|---|---|
| Distilled Water | q.s. to 100% | q.s. to 100% |
| Sodium lauroyl glutamate (Galsoft SLGL (30%) | 18.0% | 18.0% |
| Sodium cocoyl glutamate and sodium cocoyl isethionate (2:1, powder) (Galsoft GLI 21) | 6.0 % | 6.0% |
| Galaxy CAPB (35 % solids) | 13.0% | 13.0% |
| Glycerin | 2.0% | 2.0% |
| Antimicrobial (CG or GMU or combination of the two) | q.s | q. s |
| Citric acid (50% aq. sol) | q.s. pH = 5.5 | q.s. pH = 7.0 |

**Table 2b: Preservation efficacy of the N-capryloyl L-glutamine and the glyceryl monoundecylenate**

| Sr No. | Formulations of Table 2a with *N*-Capryloyl L-glutamine and /or Glyceryl Monoundecylenate | pH | Challenge Test |
|---|---|---|---|
| 1 | *N*-Capryloyl L-Glutamine (2.0 %) | 5.5 | Passes |
| 2 | *N*-Capryloyl L-Glutamine (2.0 %) | 7.0 | Passes |
| 3 | *N*-Capryloyl L-Glutamine (1.5 %) | 5.5 | Passes |
| 4 | *N*-Capryloyl L-Glutamine (1.5 %) | 7.0 | Fails |
| 5 | Glyceryl Monoundecylenate (2.0 %) | 7.0 | Passes |
| 6 | Glyceryl Monoundecylenate (2.0 %) | 5.5 | Passes |
| 7 | Glyceryl Monoundecylenate (1.5 %) | 7.0 | Fails |
| 8 | Glyceryl Monoundecylenate (1.5 %) | 5.5 | Fails |
| 9 | *N*-Capryloyl L-Glutamine (0.75 %) + Glyceryl Monoundecylenate (0.75%) - (1:1 ratio) | 5.5 | Passes |
| 10 | *N*-Capryloyl L-Glutamine (0.75 %) + Glyceryl Monoundecylenate (0.75%) - (1:1 ratio) | 7.0 | Passes |
| 11 | *N-*Capryloyl L-Glutamine (0.6 %) + Glyceryl Monoundecylenate (0.6%) - (1:1 ratio) | 5.5 | Passes |
| 12 | *N-*Capryloyl L-Glutamine (0.6 %) + Glyceryl Monoundecylenate (0.6 %) - (1:1 ratio) | 7.0 | Passes |
| 13 | *N-*Capryloyl L-Glutamine (1.0 %) + Glyceryl Monoundecylenate (0.5%) - (2:1 ratio) | 5.5 | Passes |
| 14 | *N-*Capryloyl L-Glutamine (1.0 %) + Glyceryl Monoundecylenate (0.5%) - (2:1 ratio) | 7.0 | Passes |
| 15 | *N-*Capryloyl L-Glutamine (0.5 %) + Glyceryl Monoundecylenate (1.0 %) - (1:2 ratio) | 5.5 | Passes |
| 16 | *N-*Capryloyl L-Glutamine (0.5 %) + Glyceryl Monondecylenate (1.0 %) - (1:2 ratio) | 7.0 | Passes |
| 17 | *N-*Capryloyl L-Glutamine (0.5 %) + Glyceryl Monondecylenate (1.5 %) - (1:3 ratio) | 7.0 | Fails |
| 18 | *N-*Capryloyl L-Glutamine (1.5 %) + Glyceryl Monondecylenate (0.5 %) - (3:1 ratio) | 7.0 | Fails |

### Example 4: Evaluation of prebiotic /skin microbiota supporting property of mixture of (1:1 by weight) glyceryl mono undecylenate and N-capryloyl L-glutamine

The following media, microbes and test substances are required.
1) Test substance: *N-*capryloyl L-glutamine (Example1) and glyceryl monoundecylenate (Example 2)
2) Stock solutions of Test substance: 0.1% (0.05% of capryloyl glutamine and 0.05 % of glyceryl monoundecylenate), 0.3% (0.15% of capryloyl glutamine and 0.15 % of glyceryl monoundecylenate and 0.5% (0.25% of capryloyl glutamine and 0.25 % of glyceryl monoundecylenate) in distilled water.
3) Diluent used: Phosphate Buffer Saline (PBS) and Tryptic Soy Broth (TSB) pH: 5.5
4) Media used: Modified Letheen Agar
5) Redox indicator: 1% solution of Triphenyl Tetrazolium Chloride (TTC)
6) Medium used for performing dilutions: Tryptone Azolectin Tween^{™} (TAT) broth.
7) Microbe Cultures: 24h-old culture of Staphylococcus epidermidis NCIM 2493 1×10⁷ and Staphylococcus aureus ATCC 6538 1×10⁷

**Table 3a: Control and test sets of Sa (Staphylococcus aureus ATCC 6538)**

| **Sr no.** | **Set for Sa** | **PBS (ml)** | **TSB (ml)** | **Stock (µl)** | **Culture (µl)** | **TTC (µl)** |
|---|---|---|---|---|---|---|
| 1 | Control | 2.5 | 2.5 | - | 100 | 70 |
| 2 | Test Substance (0.1%) | 2.5 | 2.5 | 1000 | 100 | 70 |
| 3 | Test Substance (0.3%) | 2.5 | 2.5 | 1000 | 100 | 70 |
| 4 | Test Substance (0.5%) | 2.5 | 2.5 | 1000 | 100 | 70 |

**Table 3b: Control and test sets of Se (Staphylococcus epidermidis NCIM 2493**

| **Sr no.** | **Set for Se** | **PBS (ml)** | **TSB (ml)** | **Stock (µl)** | **Culture (µl)** | **TTC (µl)** |
|---|---|---|---|---|---|---|
| 1 | Control | 2.5 | 2.5 | - | 100 | 70 |
| 2 | Test Substance (0.1%) | 2.5 | 2.5 | 1000 | 100 | 70 |
| 3 | Test Substance (0.3%) | 2.5 | 2.5 | 1000 | 100 | 70 |
| 4 | Test Substance (0.5%) | 2.5 | 2.5 | 1000 | 100 | 70 |

**Table 3c: Effect of the preservative composition of the present invention on S. aureus and S. epidermidis.**

| | **Test Substance (*N-*capryloyl L-glutamine:glyceryl monoundecylenate :: 1:1)** | | | **Control** |
|---|---|---|---|---|
| | **0.1%** | **0.3%** | **0.5%** | |
| ***S. aureus*** | 1.4×10⁶ | 3.9×10⁵ | 1.6×10⁵ | 1.3×10⁷ |
| ***S. epidermidis*** | 1.8×10⁷ | 1.8×10⁷ | 1.1×10⁷ | 1.3×10⁷ |

### Procedure:

1) Prepare two sets of tubes: 1) for *Staphylococcus aureus* (Sa) Table 3a and 2) for Staphylococcus epidermidis (Se) (Table 3b)
2) Control: Add 2.5ml of PBS and 2.5ml of TSB and 100µl of *Staphylococcus aureus* culture and 70 µl of TTC
3) Test substance (0.1%): Add 2.5ml of PBS and 2.5ml of TSB, 100µl of Staphylococcus aureus culture, 70 µl of TTC and 1000 µl of 0.1% Test substance stock solution
4) Test substance (0.3%): Add 2.5ml of PBS and 2.5ml of TSB, 100µl of Staphylococcus aureus culture, 70 µl of TTC and 1000 µl of 0.3 % Test substance stock solution
5) Test substance (0.5%): Add 2.5ml of PBS and 2.5ml of TSB, 100µl of Staphylococcus aureus culture, 70 µl of TTC and 1000 µl of 0.5 % Test substance stock solution
6) Vortex and incubate the tubes at 35°C for 24hrs.
7) After 24 hrs, observe the results (for formation of red color due reduction of TTC to 1,3,5 Tri phenyl formazan)
8) Perform serial dilution till 10⁷ using TAT broth on the above set tubes and plate is using MLA and incubate the plates at 35°C for 48hrs.

### Example 5: Preparation of body-wash and its preservation with N-capryloyl L-glutamine (0.75%) and glyceryl monoundecylenate (0.75%)

| **Ingredients** | **Trade names/Supplier** | **Weight %** |
|---|---|---|
| **Phase A** | | |
| Water | - | To make 100.0 |
| Sodium cocoyl glutamate (30%) | Galsoft SLGL | 7.00 |
| Sodium cocoyl isethionate | Galsoft SCI 85 (N) | 3.00 |
| Lauryl polyglucoside (50%) | Galsoft EcoCare 195 | 8.00 |
| Cocoamidopropyl betaine (36%) | Galaxy CAPB | 18.00 |
| Tetrasodium glutamate diacetate | Aquacid 2014 EX | 0.30 |

| **Phase B** | | |
|---|---|---|
| Glyceryl mono laurate | Finester LG 9000 | 2.0 |
| Polyethylene glycol 150 distearate | Aculyn 60 | 1.0 |
| *N*-capryloyl L-glutamine | Example 1 | 0.75 |
| Glyceryl mono undecylenate | Example 2 | 0.75 |
| 1, 3- Propane diol distearate | - | 2.00 |

Procedure: Phase A is prepared separately by gentle stirring of the components at 60°C. The components of phase B are added one by one to stirred mass of phase A and agitation is continued at 60 °C till the mass becomes homogeneous. It is cooled to ambient temperature and pH of the stirred mass is adjusted to 5.5 with citric acid solution. After ensuring the uniformity of pH, color and fragrance are added and stirring continued till the mass becomes homogeneous. The resultant formulation has viscosity of 5000 cp at 25 °C. Using same formula another batch is made and the pH is adjusted to 7.0. Both compositions (one at pH 5.5 and pH 7.0) pass the preservation efficacy test. The composition of body-wash at pH 5.5, after one month at 40°C is rechallenged for the preservation efficacy and it does clear the preservation efficacy test.

### Example 6: Preparation of `Sulphate-Free' shampoo and its preservation with combination of N-capryloyl L-glutamine and glyceryl monoundecylenate.

| **Ingredients** | **Trade name/Supplier** | **Weight %** |
|---|---|---|
| **Phase A** | | |
| Water | - | To make 100.0 |
| Sodium cocoyl sarcosinate (30%) | Galsoft NaLS | 5.0 |
| Sophorolipids (50%) | GalSophorin | 7.0 |
| Sodium cocoyl taurate (40%) | Galsoft SCT 40 (LPH) | 15.0 |
| Cocamidopropyl betaine (36%) | Galaxy CAPB | 10.0 |
| Lauryl polyglucoside (50%) | Galsoft EcoCare 195 | 15.0 |
| Glycerine | - | 2 |
| Sodium gluconate | Pallavi Equipment and Chemicals | 0.2 |
| Tetrasodium glutamate diacetate | Aquacid 2024 EX | 0.2 |

| **Phase B** | | |
|---|---|---|
| Polyethylene glycol 150 distearate | Aculyn 60 | 2.0 |
| 1,3 propane diol distearate | - | 3.0 |
| *N*-capryloyl L-glutamine | Example 1 | 0.75 |
| Glyceryl mono undecylenate | Example 2 | 0.75 |

| **Phase C** | | |
|---|---|---|
| Polyquaternium -7 (9 %) | Galsilk-7 | 3.0 |

Procedure: Phase A is prepared separately by gentle stirring of the components at 60°C. The components of phase B are added one by one to the stirred mass of phase A and agitation is continued at 60°C till the mass becomes homogeneous. The first component of phase C is added. It is cooled to ambient temperature and pH of the stirred mass is adjusted to 5.5 with caustic lye solution. After ensuring the uniformity of pH, optional color and fragrance are added and stirring continued till the mass becomes homogeneous. The resultant formulation has viscosity of 2000 cps at 25 °C. The composition described above passes the challenge test (preservation efficacy test).

### Example 7: Preparation of `extra mild' body-wash and its preservation with N-capryloyl L-glutamine and glyceryl monoundecylenate

| **Ingredients** | **Trade Name/Supplier** | **Weight %** |
|---|---|---|
| **Phase A** | | |
| Water | - | To make 100.0 |
| Sophorolipids (50%) | - | 5.0 |
| Sodium cocoyl glutamate (30%) | Galsoft SLGL | 20.0 |
| Sodium cocoyl isethionate | Galsoft SCI 85 (N) | 3.0 |
| Cocoamidopropyl betaine (36%) | Galaxy CAPB | 10.0 |
| Tetra sodium glutamate diacetate | Aquacid 2014 EX | 0.2 |

| **Phase B** | | |
|---|---|---|
| *N*-capryloyl L-glutamine | Example 1 | 0.75 |
| Glyceryl mono undecylenate | Example 2 | 0.75 |
| Polyethylene glycol 150 distearate | Aculyn 60 | 1.0 |
| Glyceryl mono laurate | Fine Organics | 2.0 |

| **Phase C** | | |
|---|---|---|
| Sodium Chloride | - | 1.5 |

Procedure: Phase A is prepared separately by gentle stirring of the components at 60°C. The components of phase B are added one by one to the stirred mass of phase A and agitation is continued at 60°C till the mass becomes homogeneous. The first component of phase C is added. It is cooled to ambient temperature and pH of the stirred mass is adjusted to 6.0 with citric acid solution. After ensuring the uniformity of pH, optional color and fragrance are added and stirring continued till the mass becomes homogeneous. The resultant formulation has viscosity of 4700 cp at 25 °C. This composition passes the challenge test for preservation.

### Example 8: Preparation of facewash and its preservation with N-capryloyl L-glutamine and glyceryl monoundecylenate

| **Ingredients** | **Trade name/Supplier** | **Weight %** |
|---|---|---|
| **Phase A** | | |
| Water | - | To make 100.0 |
| Sodium cocoyl taurate (40%) | Galsoft SCT 40 (LPH) | 27.00 |
| Cocamidopropyl betaine (36%) (without preservativie) | Galaxy CAPB | 22.00 |
| Glycerine | VVF | 5.00 |
| Sodium gluconate | Pallavi Equipments and Chemicals | 0.20 |
| Tetrasodium glutamate diacetate | Aquacid 2014 EX | 0.2 |

| **Phase B** | | |
|---|---|---|
| *N*-capryloyl L-glutamine | Example 1 | 0.75 |
| Glyceryl monoundecylenate | Example 2 | 0.75 |
| PEG-120 methyl glucose dioleate | Glucamate^{™} DOE-120 | 0.50 |
| Glyceryl monolaurate | Fine Organics | 2.0 |

| **Phase C** | | |
|---|---|---|
| Lactic acid solution (88% w/w solution) | - | To adjust pH to 4.5 |

Procedure: Phase A is prepared separately by gentle stirring of the components at 60°C. The components of phase B are added one by one to the stirred mass of phase A and agitation is continued at 60°C till the mass becomes homogeneous. It is cooled to ambient temperature and pH of the stirred mass is adjusted to 4.5 with lactic acid solution. After ensuring the uniformity of pH stirring continued till the mass becomes homogeneous. The resultant formulation has viscosity of 6400 cp at 25 °C. This composition passes the preservation efficacy test.

### Example 9: Preparation of intimate feminine hygiene wash and its preservation with a combination of N-capryloyl L-glutamine and glyceryl monoundecylenate.

| **Ingredients** | **Trade Name/Supplier** | **Weight %** |
|---|---|---|
| Water | - | To make 100.0 |
| Lauryl polyglucoside (50%) | Galsoft EcoCare 195 | 10.00 |
| Sodium lauroyl taurate and sodium lauroyl methyl isethionate (1:2 ratio), 30 % solution | Galsoft TLMI:12 | 40.00 |
| Cocoamidopropyl betaine (36%) | Galaxy CAPB | 10.00 |
| *Blend of N*-capryloyl L-glutamine and glyceryl monoundecylenate (60 % active) | Example 14 | 2.5 |
| Tetrasodium glutamate diacetate | Aquacid 2014 EX | 0.2 |
| Lactic acid solution (90 % w/w solution) | Purac Hipure 90 | To adjust pH 3.8 |

Procedure: All surfactants (first three) are added together and stirred gently at room temperature. To this stirred mass *N-*Capryloyl L-glutamine, glyceryl mono undecylenate and sodium gluconate are added. After achieving a homogeneous solution, pH is adjusted to 3.5 with lactic acid. Optionally color and fragrance are added and blended until homogenous mass is obtained. The resultant formulation has viscosity of 1075 cp at 25 °C. This composition passes the challenge test.

### Example 10: Preparation of cream and its preservation with combination of N-capryloyl L-glutamine and glyceryl monoundecylenate

| **Ingredients** | **Trade names/Supplier Name** | **Weight %** |
|---|---|---|
| **Phase A** | | |
| Water | - | To make 100.0 |
| Glycerine | VVF | 4.0 |
| Polyethyleneglycol-7-glyceryl cocoate | Galaxy PEG-7 GC | 2.0 |
| Sodium gluconate | Pallavi Equipments and Chemicals | 0.2 |
| Tetrasodium glutamate diacetate | Aquacid 2014 EX | 0.2 |

| **Phase B** | | |
|---|---|---|
| Cetostearyl alcohol | VVF LLC | 7.0 |
| Stearic acid | Emery Oleochemicals | 2.0 |
| Glyceryl monostearate | Fine Organics | 5.0 |
| Isopropyl myristate | Vajrachem | 2.5 |
| *N*-Capryloyl L-Glutamine | Example 1 | 0.5 |
| Glyceryl monoundecylenate | Example 2 | 0.5 |

Procedure: The contents of Phase A and Phase B are heated separately up to 75°C, with stirring. Phase B is then added to Phase A with constant stirring and homogenized (Silverson) for 30 minutes until emulsion is obtained. The cream is then cooled to room temperature and stirring is continued until uniform consistency is obtained. pH of the final formulation is adjusted to 6.0 with caustic lye and optional fragrance and color are blended uniformly. The resultant formulation has viscosity of 5000 cp at 25 °C. The composition passes the preservation efficacy test.

### Example 11: Preparation of antiperspirant and deodorant stick with N-capryloyl L-glutamine and glyceryl monoundecylenate

| **Ingredients** | **Trade Name/Supplier** | **Weight %** |
|---|---|---|
| Paraffin Light Liquid | Vajrachem | 38.00 |
| Bees Wax | Arjun Beeswax | 31.00 |
| Paraffin Wax | Golden Dyechem | 11.00 |
| Shea Butter | Samala Udyog Pvt. Ltd. | 11.00 |
| Cetaryl alcohol | VVF | 06.00 |
| *N*-Capryloyl L-glutamine | Example 1 | 0.3 |
| Glyceryl monoundecylenate | Example 2 | 0.3 |
| Vitamin E acetate | Matrix Life science Pvt. Ltd. | 0.9 |
| Dimethicone | Dow | 1.5 |

Procedure: All ingredients are heated together to 75 to 80 °C and gently mixed till a homogenous mass is formed. It is cooled to 60 °C blended to get uniform mass which is then added to the cast and allowed to cool to room temperature.

### Example 12: Preparation of water-less shower oil containing dermapurifiers, N-capryloyl L-glutamine (0.25 %) and glyceryl monoundecylenate (0.25 %) (US PATENT 11771643, based on Example 10)

| **Ingredients** | **Trade Name/Supplier** | **Weight %** |
|---|---|---|
| **Phase A** | | |
| Potassium cocoyl prolinate (100%) | Galsoft CoProK | 16.00 |
| Polyglyceryl 3-oleate | Fine Organics | 30.00 |
| *N*-capryloyl L-glutamine | Example 1 | 0.25 |
| Glyceryl monoundecylenate | Example 2 | 0.25 |

| **Phase B** | | |
|---|---|---|
| Soyabean oil | AAK India Pvt. Ltd. | 50.00 |
| Glyceryl monolaurate | Finester LG 9000 | 3 |
| Vitamin E acetate | Matrix lifescience Pvt. Ltd. | 0.5 |

Phase A ingredients are mixed and stirred at room temperature until homogeneous. Phase B is separately made by mixing all ingredients at room temperature. Phase B is added to Phase A under stirring till the mixture acquires homogeneity.

### Example 13 : Preparation of anti-odor skin hygiene cleansing bar with N-capryloyl L-glutamine and glyceryl monoundecylenate

To a stirred mixture of hydrogenated soyabean seed oil (360 g, 0.45 gmol), and glycerin (40 g, 0.43 gmol) at 90-95 °C under nitrogen blanket in a jacketed sigma blender (with 'Z' type blades that rotate in opposite directions to each other), sodium cocoyl isethionate (powder 85 % active, particle size < 250 µm, 450 g, 1.10 gmol) is added slowly (about two hours) and stirring is continued for three hours. The mass in the sigma blender is cooled to 65 °C under continuous kneading and to it, L-Glutamic acid, *N, N*-diacetic acid, tetrasodium salt, (2.0 g), Vitamin E acetate (5.0 g), polyglyceryl -3 oleate (90 g, 0.12 gmol), *N*-capryloyl L-glutamine (5.0 g), glyceryl monoundecylenate ( 5.0 g) and water (50.0 g) are added and the kneading operation is continued at 60-65 °C for 4 hours. The mass is then cooled to 30 to 35 °C (993 g) while continuously kneading, refined using a triple roll mill and extruded using a duplex plodder (38-42 °C) to get the billets for stamping.

Analysis of the bar cake:

| | |
|---|---|
| Moisture | 4.32 % |
| pH (5 % dispersion in water) | 5.5 |
| Active matter (as sodium cocoyl isethionate, MW 347) | 38.01% |
| Hardness | 8 to 9 |
| Grit | Absent |
| Mush | 6.0 g /50 cm² |

### Example 14 : Preparation of a blend of N-capryloyl L-glutamine and glyceryl monoundecylenate in 1:1 ratio by weight.

*N-*capryloyl L-glutamine (32g, 0.12 gmol ) is added to stirred water (28.5 mL) at 55 °C. and after complete addition of the same pH of the dispersion is adjusted to 5.5 by adding caustic solution (48 %, 7.5g). To this stirred aqueous dispersion at pH 5.5 at 55 °C, glyceryl monoundecylenate (32g, 0.12 gmol) is added and stirred for one hour.

It is cooled under stirring to room temperature to give pale yellow viscous translucent (100g) product and with pH (of 1 % dispersion in water) 4.70 and solids content of 65.5 %.

### Example 15 : Preparation of a moisturising cream using the blend N-capryloyl L-glutamine and glyceryl monoundecylenate

| **Ingredients** | **Trade name/Supplier** | **Weight %** |
|---|---|---|
| **Phase A** | | |
| Water | - | To make 100.0 |
| 1,3 - Propanediol | - | 2.0 |

| **Phase B** | | |
|---|---|---|
| Glyceryl monostearate, cetaryl alcohol and sodium steraoryl lactylate | Galfusion Bio-base | 5.0 |
| Caprylic capric triglyceride | Galmol CCT | 5.0 |
| Sunflower Seed Oil | AAK India Pvt Ltd. | 10.0 |
| Shea Butter | Samala Udyog Pvt Ltd. | 5.0 |

| **Phase C** | | |
|---|---|---|
| *N*-Capryloyl L-glutamine and glyceryl monoundecylenate (65 % active) | Example 14 | 1.85 |
| Vitamin E | Matrix life science Pvt. Ltd. | 0.1 |
| Citric acid (50%) | - | q.s. pH = 5.5 - 5.6 |

Procedure: The contents of Phase A and Phase B are heated separately up to 60°C, with stirring. Phase B is then added to Phase A with constant stirring and homogenized (Silverson) for 30 minutes until emulsion is obtained. The cream is then cooled to room temperature and Phase C is added and stirring is continued until uniform consistency is obtained. pH of the final formulation is adjusted to 5.5 with citric acid. The resultant formulation has viscosity of 7560 cps at 25 °C. The composition passes the preservation efficacy test.

## Claims

1. A personal care composition comprising:
i. *N*-capryloyl L-glutamine of Formula I;
ii. glyceryl monoundecylenate of Formula II; and 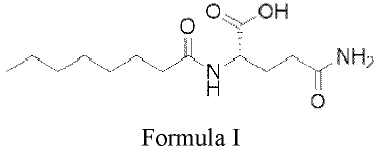 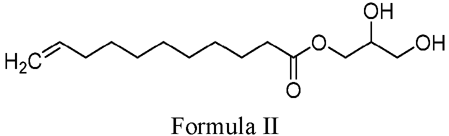
iii. one or more personal care ingredient,
wherein the ratio of the *N*-capryloyl L-glutamine of Formula I and the glyceryl monoundecylenate of Formula II, is from 1:2 to 2:1 by weight.

2. The personal care composition as claimed in claim 1, wherein weight percentage of the N-capryloyl L-glutamine of Formula I and the glyceryl monoundecylenate of Formula II is 0.5 to 2.0 of the personal care composition.

3. The personal care composition as claimed in claim 1 or 2, wherein the composition has pH ranging from 3.0 to 7.0.

4. The personal care composition as claimed in any one of claims 1 to 3, wherein the personal care composition is selected from shampoo, body wash, face wash, hair conditioner, hair serum, soap bar, syndet bar, cream, lotion, hygiene wash, deodorant, anti-perspirant and gel.

5. The personal care composition as claimed in any one of claims 1 to 4, wherein the one or more personal care ingredient is selected from anionic surfactants, amphoteric surfactants, non-ionic surfactants, cationic surfactants, pearlizers, opacifiers, emollients, anti-inflammatory, anti-microbial preservatives, UV-absorbers, UV-blockers, synthetic and/or natural polymeric conditioners, silicones, gums, rheology modifiers, polymeric rheology modifiers, film formers, vitamins, protein derivatives, anti-acne agents, anti-dandruff agents, moisturizers, humectants, emulsifiers, chelating agents, anti-oxidants, skin care active, hair active, water and mixture thereof.
